**Europäisches Patentamt**

**European Patent Office**

**Office européen des brevets**

(19)

(11) Veröffentlichungsnummer: **0 304 770 B1**

(12) **EUROPÄISCHE PATENTSCHRIFT**

(45) Veröffentlichungstag der Patentschrift: **27.12.91**

(51) Int. Cl.⁵: **C07C 31/12**, C07C 29/82

(21) Anmeldenummer: **88113269.0**

(22) Anmeldetag: **16.08.88**

(54) Verfahren zur destillativen Reinigung von rohem sec-Butylalkohol.

(30) Priorität: **26.08.87 DE 3728428**

(43) Veröffentlichungstag der Anmeldung:
**01.03.89 Patentblatt 89/09**

(45) Bekanntmachung des Hinweises auf die
Patenterteilung:
**27.12.91 Patentblatt 91/52**

(84) Benannte Vertragsstaaten:
**AT BE CH DE ES FR GB GR IT LI NL SE**

(56) Entgegenhaltungen:
**EP-A- 0 051 164**
**DE-A- 2 033 707**
**DE-B- 1 017 602**
**DE-B- 2 429 770**
**GB-A- 829 424**

(73) Patentinhaber: **RWE-DEA Aktiengesellschaft
für Mineraloel und Chemie
Überseering 40
W-2000 Hamburg 60(DE)**

(72) Erfinder: **Osterburg, Günther
Ahornstrasse 5
W-4137 Rheurdt(DE)**

(74) Vertreter: **Schupfner, Gerhard D.
Müller, Schupfner & Gauger Karlstrasse 5
Postfach 14 27
W-2110 Buchholz/Nordheide(DE)**

**Beschreibung**

Die vorliegende Erfindung betrifft ein Verfahren zur kontinuierlichen destillativen Reinigung von rohem sec-Butylalkohol, der durch katalytische Hydratisierung von n-Butenen bei erhöhter Temperatur und erhöhtem Druck und Abtrennung von nichtumgesetztem Olefin aus dem Reaktionsprodukt erhalten wurde, durch Einleitung des Rohalkohols in den oberen Teil einer Trennkolonne, Zuführung der zur Verdampfung notwendigen Energie durch einen Aufkocher am Bodenteil der Trennkolonne, Abdestillation der azeotrop und leichtsiedenden Nebenprodukte über Kopf in Gegenwart von Wasser und Abziehung des vorgereinigten sec-Butylalkohols am Sumpf der Trennkolonne und anschließende Abtrennung der schwersiedenden Nebenprodukte.

Aus der DE-PS 24 29 770 ist ein Verfahren zur kontinuierlichen Herstellung von niederen Alkoholen durch direkte katalytische Hydratisierung dampfförmiger niederer Olefine mit flüssigem Wasser in Gegenwart von Säuren oder stark sauren Feststoffen bekannt, bei dem der gebildete Alkohol dampfförmig zusammen mit überschüssigem Reaktionsgas am Kopf des Reaktors abgezogen, durch partielle Entspannung und gegebenenfalls durch zusätzliche Abkühlung von gasförmigen Restolefinen abgetrennt und in Form eines über 80%igen Alkohols gewonnen wird. Nach diesem Verfahren kann der Alkohol sowohl durch Zwischenentspannung in einem Abscheidersystem als auch durch Abtrennung in einer in bekannter Weise arbeitenden Druckkolonne gewonnen werden. In beiden Fällen wird z.B. ein sec-Butylalkohol mit einem 15- bis maximal 23%igen Wasseranteil erhalten.

DE-PS 30 40 997 beschreibt ein Verfahren zur kontinuierlichen Herstellung von im wesentlichen wasserfreiem sec-Butylalkohol durch katalytische Hydratation von n-Butenen in Gegenwart eines stark sauren Kationenaustauschers als Katalysator, bei dem man das am Kopf des Reaktors dampfförmig abgeführte Reaktionsprodukt im Druckbereich von 2 bis 60 bar und bei Temperaturen von höchstens 135°C verflüssigt, in einem Abscheider das im Reaktionsprodukt enthaltene Wasser abtrennt, das flüssige Alkohol-Restgasgemisch verdampft und in einer Druckkolonne bei einem Druck von 3 bis 30 bar auftrennt und trockenen sec-Butylalkohol mit weniger als 0,1 % Wasser gewinnt.

Für die Entwicklung eines Destillationsverfahrens zur Reinigung eines rohen Syntheseproduktes sind neben der Art, der Menge und den Eigenschaften der darin enthaltenen Nebenprodukte auch die Reinheitsforderungen an das zu erzeugende Hauptprodukt und die Effektivität der Trennschritte von Bedeutung. Bei einem sec-Butylalkohol (SBA), der überwiegend zur Erzeugung von Methylethylketon (MEK) durch katalytische Dehydrierung eingesetzt wird, ist die Forderung nach Reinheit des Alkohols insbesondere auch im Zusammenhang mit Auswirkungen bei der MEK-Synthese bzw. MEK-Destillation zu beachten.

Die Art der in einem durch katalytische Hydratation von n-Butenen in Gegenwart von stark sauren Kationenaustauschern erzeugten sec-Butylalkohol enthaltenen Nebenprodukte ist zum großen Teil identisch mit denen aus konventionellen sec-Butylalkoholsynthesen etwa mittels Schwefelsäure. Sie entstehen durch synthesebedingte Neben- und Folgereaktionen von n-Butenen, aber auch durch Abreagieren von typischen, im Syntheseeinsatz enthaltenen Begleitstoffen wie u.a. Isobuten, Propen und 1,3-Butadien.

Kennzeichnend für die Nebenproduktbildung bei der katalytischen Hydratation von n-Butenen in Gegenwart von stark sauren Kationenaustauschern als Katalysator ist die erheblich geringere Neigung zur Oligomerisierung von n-Butenen im Vergleich zur konventionellen Synthese. In der Regel werden nur dimere Folgeprodukte, aber keine höheren Polymeren wie beim Schwefelsäureverfahren gebildet.

Dieses gilt insbesondere auch für das im Syntheseeinsatz in unterschiedlichen Mengen enthaltene Isobuten bzw. Propen, das vorzugsweise zu tert-Butylalkohol bzw. Isopropylalkohol hydratisiert wird.

Weiterhin ist für diese Synthese typisch, daß die Bildung von Di-sec-butylether deutlicher von den Synthesebedingungen beeinflußt wird und dieser Äther deshalb im Syntheseverlauf in unterschiedlichen Mengen anfallen kann. So können in einem solchen rohen sec-Butylalkohol als Hauptnebenprodukte wechselnde Mengen an tert-Butylalkohol (TBA), Isopropylalkohol (IPA) und Di-sec-butylether (DSBE) enthalten sein.

Die in der Tabelle I aufgeführten Produkte kennzeichnen in ihrer Menge und in ihrem Trennverhalten gegenüber secButylalkohol (bzw. auch gegenüber MEK als Folgeprodukt) die bei der Synthese entstehenden und für ein Reinigungsverfahren maßgeblichen Nebenprodukte im sec-Butylalkohol. Dabei sind wechselnde Mengen an polaren und unpolaren Nebenprodukten nicht ohne Bedeutung für die Konzeption eines Reinigungsverfahrens.

Tabelle I

|  | kp $^\circ$ C, 1013 mbar |
|---|---|
| 1. Di-sec-butylether | 121 |
| 2. tert-Butylalkohol | 82,5 |
| 3. Dimere $C_4$-Kohlenwasserstoffe | 99 - 135 |
| 4. Isopropylalkohol | 82,4 |
| 5. n-Butylalkohol | 117,4 |
| 6. Buten-(2)-ol-(1), cis-Crotonalkohol | 123,6 |
| trans-Crotonalkohol | 121,2 |
| 7. $C_8$-Alkohol | 160 |

Für die Reinheit des zu erzeugenden sec-Butylalkohols ist besonders die quantitative Abtrennung der Nebenprodukte, die nicht nur die Reinheit von sec-Butylalkohol, sondern vor allem die Weiterverarbeitung zu MEK und dessen Reinheit beeinträchtigen könnten, von Bedeutung.

Hierzu zählt:

1. Die Abtrennung von Di-sec-butylether als Hauptnebenprodukt von sec-Butylalkohol.

2. Die Abtrennung aller gesättigten und ungesättigten $C_8$-Kohlenwasserstoffe. Dies gilt auch für die schwer abtrennbaren höher siedenden Oktene und für die von 1,3-Butadien abzuleitenden und bei 135 $^\circ$ C siedenden Oktadiene. Beide Produktgruppen, die mit MEK azeotrop nicht flüchtig sind, werden bei der Dehydrierung von sec-Butylalkohol durch Hydrierung in mit MEK azeotrop flüchtige Substanzen umgewandelt.

3. Die Abtrennung von tert-Butylalkohol und Isopropylalkohol, die von MEK nur schlecht oder nicht abgetrennt werden können.

4. Die Abtrennung von n-Butylalkohol, der bei der MEK-Synthese zu Butyraldehyd dehydriert wird und danach erhebliche Reinigungsprobleme mit sich bringt.

5. Die Abtrennung aller höher als n-Butylalkohol siedenden Produkte, die bei der Dehydrierung von sec-Butylalkohol im Gasphasenverfahren schon in geringen Mengen zur Inaktivierung des MEK-Katalysators führen.

Wie die Tabelle II an ausgewählten Beispielen zeigt, sind die azeotropen Eigenschaften von sec-Butylalkohol mit einer Reihe von Nebenprodukten, oder diese untereinander, derart kompliziert, daß die Abtrennung bestimmter Nebenprodukte nur in Form der ternären Azeotrope unter Zuhilfenahme von Wasser gelingt.

Tabelle II

| Binäre und ternäre azeotrope Daten aus dem Bereich der SBA-Destillation: | | | | | | |
|---|---|---|---|---|---|---|
| Nr. | Komponenten | | | Azeotrop kp $^\circ$ C, 1013 mbar | Azeotrop in Gew.% | | |
| | A | B | C | | A | B | C |
| 1. | SBA | DSBE | - | 99,0 | 77,0 | 23,0 | - |
| 2. | SBA | DSBE | $H_2O$ | 84,4 | 34,1 | 47,9 | 18,0 |
| 3. | DSBE | - | $H_2O$ | 87,5 | 72,2 | - | 27,8 |
| 4. | SBA | Okten[+] | - | 95,4 | 62,5 | 37,5 | - |
| 5. | TBA | Okten[+] | - | 81,6 | 90,6 | 9,4 | - |
| 6. | TBA | DSBE | $H_2O$ | 77,9 | 53,7 | 32,3 | 14,0 |
| 7. | TBA | - | $H_2O$ | 79,9 | 88,2 | - | 11,8 |

[+]) Okten = 3,4-Dimethyl-hexen-(2), kp $^\circ$ C 114 $^\circ$ C

So werden dem Stand der Technik entsprechend wasserhaltige Rohalkohole aus konventionellen Hydratationsprozessen destillativ unter Zuhilfenahme des darin enthaltenen Wassers zunächst von den unter diesen Umständen flüchtigen Substanzen befreit, bevor das Wasser von sec-Butylalkohol abgetrennt und der trockene sec-Butylalkohol von den restlichen höher siedenden Verunreinigungen abgetrennt wird.

In einem seit Jahrzehnten großtechnisch durchgeführten Verfahren wird z.B. roher sec-Butylalkohol, der

EP 0 304 770 B1

nach dem Abtreiben von Schwefelsäure mit einem Wasseranteil von 30 bis 50 % anfällt, zusätzlich mit Wasser verdünnt, ehe aus diesem Dünnalkohol die gegenüber sec-Butylalkohol niedriger siedenden Produkte oder azeotrop niedriger siedende Produktgemische in einer sogenannten Vorreinigungskolonne abdestilliert werden. Diese zusätzliche Wasserverdünnung des Rohalkohols bewirkt, daß durch eine quasi-Extraktivdestillation die relative Flüchtigkeit der azeotrop siedenden Nebenprodukte gegenüber dem in Wasser gelösten sec-Butylalkohol erhöht und damit die Trennleistung bzw. der Trenneffekt in der vorgegebenen Vorreinigungsstufe verstärkt wird.

Die Erhöhung des Trenneffekts steigt mit der Wasserverdünnung, deren Begrenzung aus wirtschaftlichen Gründen bei ca. 90 % liegt. Von Nachteil bei dieser Wasserverdünnung ist es, daß die Erhöhung der relativen Flüchtigkeit nur auf die Abtrennung von wasserunlöslichen Nebenprodukten wie Ether und Kohlenwasserstoffe zutrifft, während die Ab-Abtrennung wasserlöslicher und leichtsiedender Nebenprodukte wie Isopropylalkohol und tert-Butylalkohol dadurch teilweise oder ganz verhindert wird.

Weiterhin ist bei dieser Maßnahme von Nachteil, daß die hierfür notwendige große Wassermenge in zwei Trennstufen, Azeotropierungs- und Absolutierungskolonnen, wieder von sec-Butylalkohol abgetrennt werden muß. Erst danach können in einer vierten Destillationskolonne die leichtsiedenden wasserlöslichen Nebenprodukte aus trockenem sec-Butylalkohol abdestilliert werden, bevor in der fünften und letzten Destillationsstufe die gegenüber sec-Butylalkohol höher und nicht azeotrop siedenden Nebenprodukte als Schwersieder abgetrennt werden.

Aus dieser vereinfachten Beschreibung wird deutlich, daß das Hauptproblem bei der destillativen Reinigung von sec-Butylalkohol ist, eine geeignete Lösung zur Abtrennung von Leichtsiedern, Azeotropbildnern und Wasser zu finden.

Es sind zwar Verfahren bekannt geworden, denen eine Vereinfachung des Destillationsprozesses zur Reinigung von sec-Butylalkohol zugrunde liegt. Bei diesen Verfahren wird jedoch wasserhaltiger Rohalkohol eingesetzt:

So wird bei dem Verfahren der DE-AS 1 017 602 aus dem Jahre 1955, gleichlautend mit der US-PS 28 75 138, eine nur zweistufige Destillation zur Reinigung von sec-Butylalkohol vorgeschlagen, bei der aus einem Rohalkohol, der zuerst mit genügend Wasser verdünnt wird, in einer ersten Kolonne niedrigsiedende Verunreinigungen (einschließlich der azeotrop siedenden Verunreingiungen) und Wasser gemeinsam abgetrennt werden. Im Anschluß daran wird in einer zweiten Kolonne eine Nachtrennung von niedrigsiedenden und eine Abtrennung von hochsiedenden Verunreinigungen durchgeführt, wobei der sec-Butylalkohol seitlich aus der Kolonne abgeführt wird. Bei diesem Verfahren ist erforderlich, daß in der Rohalkoholbeschickung genügend Wasser vorhanden ist, um alle unter 99,5 °C siedenden Verunreinigungen und Azeotrope abzudestillieren.

Eine für die Abtrennung von azeotrop siedenden Verunreinigungen gemäß diesem Verfahren wichtige Forderung ist, daß der sec-Butylalkoholgehalt im Kopfprodukt der ersten Kolonne im Überschuß auf die mögliche ternäre Zusammensetzung des azeotropen Gemisches vorhanden sein soll.

Unter den angegebenen Ausführungen und Bedingungen des Verfahrens der DE-AS 10 17 602 wird jedoch nicht die angestrebte hohe Reinheit des sec-Butylalkohols erreicht.

In der DE-OS 2 033 707 wird die destillative Reinigung eines wasserhaltigen sec-Butylalkohols unter Verwendung einer einzigen Destillationskolonne beschrieben. Diese Veröffentlichung basiert in ihrem wesentlichen Inhalt auf der Lehre der vorgenannten DE-AS 1 017 602 bzw. US-PS 2 875 138. Als erfindungswesentlich wird der Abzug von dampfförmigem sec-Butylalkohol aus dem unteren wasserfreien Kolonnenteil herausgestellt.

Der Fachmann vermag in dieser Anmeldung kein wirtschaftlicheres und vor allem effektiveres Verfahren zur Reinigung von sec-Butylalkohol erkennen. Letzten Endes wurden nur die aus der DE-AS 1 017 602 bekannten beiden Kolonnen aufeinandergestellt, ohne daß die hier erzielte Schwersiederreinheit in dem im unteren Kolonnenteil abgezogenen dampfförmigen sec-Butylalkohol erreicht bzw. die in der zweiten Kolonne erforderliche Verdampfung des gesamten sec-Butylalkohols vermieden wird. Daneben wird auch nur eine unzureichende Abtrennung von Di-sec-butylether in dem erzeugten sec-Butylalkohol erreicht.

In der GB-PS 829 424 wird ein Destillationsverfahren zur Reinigung von wasserhaltigem sec-Butylalkohol aus einem konventionellen Hydratationsprozeß beschrieben, bei dem ebenfalls in einer (ersten) Kolonne 2 die azeotrop siedenden Nebenprodukte gemeinsam mit Wasser und in einer (zweiten)Kolonne 3 die höher siedenden Nebenprodukte vom sec-Butylalkohol abgetrennt werden.

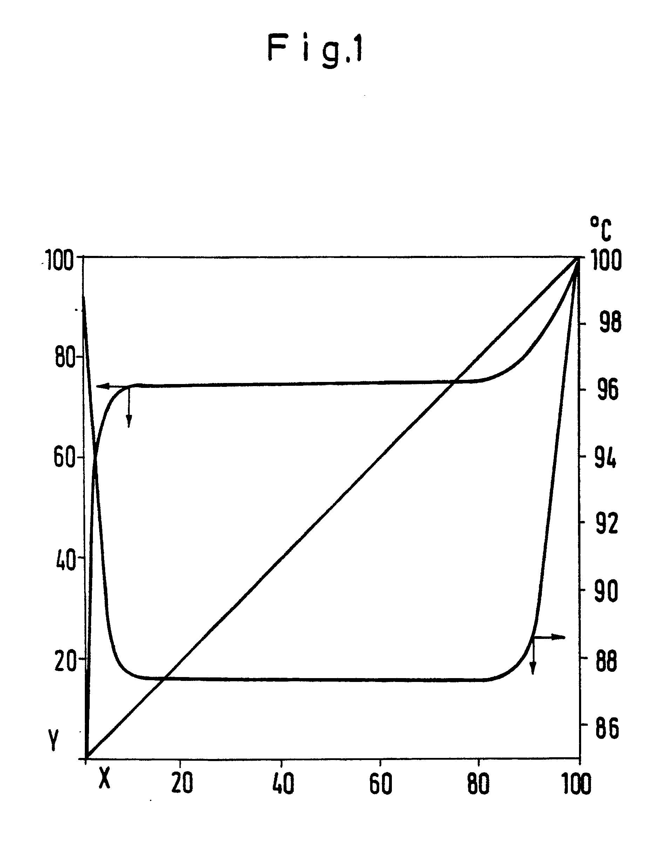

Wie in Figur 1 dieser britischen Patentschrift dargestellt, wird dieses Verfahren durch die Aufarbeitung der anfallenden Nebenproduktströme in einem Extraktor 18 und Kolonne 1 ergänzt.

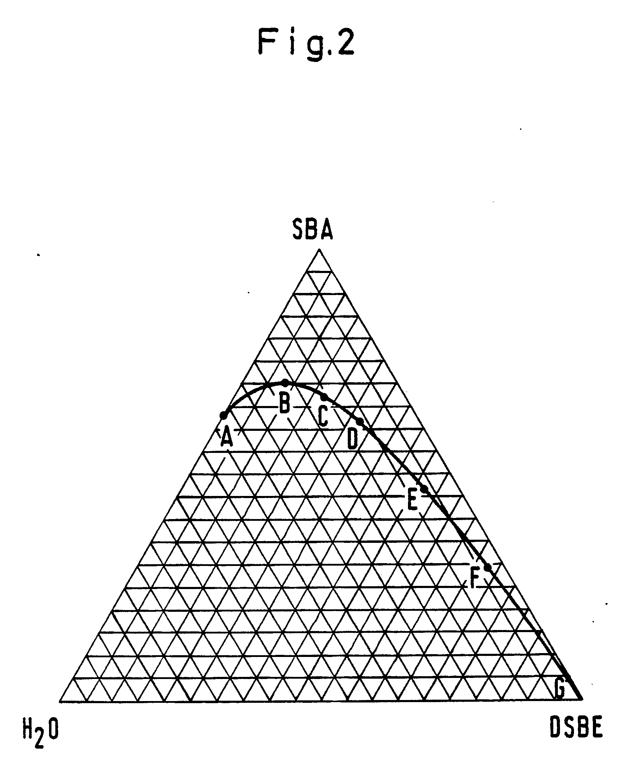

Wesentlicher Inhalt dieser Patentschrift sind jedoch die in Figur 2 dargestellten technischen Durchführungsverbesserungen an Kolonne 2, die die komplizierte Einstellung der für diese Trennaufgabe geforderten Gleichgewichtslage in der Kolonne gewährleisten sollen. Aufgrund der dort beschriebenen Maßnahmen soll

4

sich die Einhaltung des thermodynamischen Gleichgewichts in Kolonne 2 in erster Linie an der Abtrennung des mit der Rohalkoholbeschickung eingebrachten Wassers orientieren. Dazu wird zum einen bei vorgegebener Energiezufuhr zum Reboiler und einer vorgegebenen Rücklaufmenge über Strom 9 die Menge der Rohalkoholbeschickung und damit der Wassereintrag über Strom 4 in Abhängigkeit zur Temperatur am Boden 12 geführt. Die Temperatureinhaltung am Boden 12 soll gewährleisten, daß kein Wasser in das Sumpfprodukt der Kolonne 2 gelangt. Zum anderen soll durch kontrollierte Rückführung einer etherreichen organischen Phase (Strom 24) in die Rohalkoholbeschickung und einer wasserreichen Phase (Strom 25) auf den Boden 32 in Abhängigkeit zur Temperatur am Boden 28 die Austrocknung der Kolonne nach oben hin verhindert werden.

Es wird also eine Änderung des Wasserhaushalts in der Kolonne, mindestens zwischen dem 12. und 28. Boden, hingenommen. Es kommt bei dieser Verfahrensbeschreibung nicht zum Ausdruck, daß alle diese komplizierten Regelungen zur Gleichgewichtseinstellung in der Kolonne auch durch die Zusammensetzung des Kopfproduktes aus der Kolonne 2 und des daraus resultierenden Löslichkeitsproduktes im Abscheider 7 in starkem Maße beeinflußbar sind und damit nicht einfacher werden.

Auch bei diesem Verfahren muß die Reinheit des erhaltenen sec-Butylalkohols unzureichend sein. Es werden dort auch keine Angaben gemacht, die eine Beurteilung der Qualität des erhaltenen sec-Butylalkohols gestatten.

Die in den vorgestellten Veröffentlichungen beschriebenen Verfahren lassen Bedenken an deren Zweckmäßigkeit bzw. Durchführbarkeit und an deren Trenneffektivität zur Erzielung der geforderten Produktreinheit aufkommen. Sie sind darüber hinaus wegen des bei der Durchführung geforderten hohen Wassergehaltes im Rohalkohol grundsätzlich nicht zur Reinigung eines trockenen sec-Butylalkohols geeignet.

Der vorliegenden Erfindung liegt die Aufgabe zugrunde, ein geeignetes Destillationsverfahren zur Reinigung eines durch katalytische Hydratation von n-Butenen, insbesondere in Gegenwart von stark sauren Kationenaustauschern erzeugten, durch Wasserabscheidung aus dem verflüssigten Reaktionsprodukt getrockneten und durch Abtrennung vom Restgasgemisch durch Verdampfung in einer Druckkolonne isolierten sec-Butylalkohol zu finden, ohne Verzicht auf den durch die Wasserfreiheit in dem so erzeugten sec-Butylalkohol gegebenen Vorteil.

Es sollte ein Destillationsverfahren zur Reinigung von trockenem rohen sec-Butylalkohol entwickelt werden, das einerseits einen sec-Butylalkohol in der gewohnt guten Qualität liefert, andererseits das alte umständliche Verfahren ersetzt bzw. die in den veröffentlichten Verfahren erkennbaren Nachteile beseitigt.

Dabei war klar, daß wegen der Ähnlichkeit der Nebenprodukte auch die Naturgesetzlichkeiten, die bei dem alten Destillationsverfahren erkannt wurden, bei der destillativen Reinigung eines nach dem Direkthydratations-Verfahren erhaltenen sec-Butylalkohols Gültigkeit haben müssen. Das heißt, auf die Anwendung von Wasser bei der Destillation kann nicht verzichtet werden. Andererseits sollte jedoch die Wasseranwendung in einer Art und Weise stattfinden, die den Wegfall von drei Destillationskolonnen des alten Verfahrens, nämlich Azeotropierungs-, Absolutierungs- und Leichtsieder-Kolonne, erlaubt.

Diese Aufgabe wird erfindungsgemäß dadurch gelöst, daß man zur Reinigung von praktisch wasserfreiem Rohalkohol dem obersten Boden der Trennkolonne die für die azeotrope Zusammensetzung des anfallenden Kopfprodukts erforderliche Wassermenge zuführt, und

durch Anlegen einer Richttemperatur im Bereich von $87,5\,°C$ bis $99,5\,°C$ an einen mittleren Boden der Trennkolonne (Temperaturführungsboden) das zugeführte Wasser in der Trennkolonne bis zu diesem Temperaturführungsboden herabführt,

den Rohalkohol auf einen Boden der Trennkolonne kurz unterhalb des Kolonnenkopfes (Zulaufboden) aufgibt, die unpolaren als ternäre Azeotrope mit SBA und Wasser leichtsiedenden Nebenprodukte in dem wasserenthaltendem Kolonnenteil zwischen Zulauf- und Temperaturführungsboden durch Extraktivdestillation azeotrop abtreibt, gleichzeitig die durch die Anwendung von Wasser in ihrer Abtrennung behinderten polaren Nebenprodukte in dem wasserfreien Kolonnenteil zwischen dem Temperaturführungsboden und dem Kolonnensumpf aus dem sec-Butylalkohol abtreibt, und diese Nebenprodukte gemeinsam in dem Kolonnenteil zwischen Zulaufboden und Kopf durch Rektifikation aufkonzentriert, das über Kopf anfallende Destillationsprodukt kondensiert und die erhaltenen zwei Phasen über einen Rücklaufbehälter in einem Strom direkt vom Boden des Rücklaufbehälters auf den obersten Boden der Trennkolonne zurückführt und lediglich den durch die Destillation des Rohalkohols bedingten Zuwachs an oberer leichter Phase als Nebenproduktstrom abzieht,

die zur Aufrechterhaltung der gewünschten azeotropen Zusammensetzung des Kopfprodukts erforderliche und in dem Nebenproduktstrom abgeführte Wassermenge durch Frischwasser ersetzt und dieses mit dem zweiphasigen Rücklauf dem obersten Boden der Trennkolonne zuführt, wobei man die Frischwasserzuführung anhand der Kopftemperatur der Trennkolonne regelt.

Nach einer bevorzugten Ausführungsform des erfindungsgemäßen Verfahrens ist der Rücklaufbehälter so als Abscheider ausgebildet, daß man nicht nur die leichte Phase als Nebenproduktstrom abführt und das zweiphasige Destillationsprodukt an der Trennlinie zwischen leichter und schwerer Phase abführt und auf den oberen Boden der Trennkolonne zurückführt, sondern auch aus dem unteren Teil des Behälters wäßrige Phase abzieht, daraus durch Azeotropdestillation sec-Butylalkohol-Wasser-Azeotrop gewinnt und dieses im Kreislauf zur Trennkolonne zurückführt.

Mit dem geregelten Wasserabzug kann man einen erhöhten Wassereintrag in die Trennkolonne ausgleichen.

Die Gesamtbodenzahl in der für die Abtrennung von leichtsiedenden und azeotrop siedenden Nebenprodukten benötigten Destillationskolonne ist nicht festgelegt, da die Trennschärfe der Kolonne außerdem vom Bodenwirkungsgrad der Trenneinbauten, von der Rücklaufbelastung bezogen auf den Kolonnendurchsatz und durch den sec-Butylalkohol-Überschuß im Kopfprodukt beeinflußt werden kann.

Man arbeitet bevorzugt in einem Bereich von insgesamt 55 bis 125 Böden, von denen 5 bis 10 Böden oberhalb des Zulaufbodens als Rektifikationsteil angeordnet und die anderen Böden durch Anlegen der Richttemperatur an den Temperaturführungsboden in einen wasserhaltigen (über dem Temperaturführungsboden) und einen trockenen Abtriebsteil (unter dem Temperaturführungsboden) aufgeteilt sind.

Eine geringere Bödenzahl würde zu einer unwirtschaftlich hohen Rücklaufbelastung und dem dadurch verursachten Energieverbrauch führen. Bei einer zu hohen Bodenzahl wird der technische Aufwand nicht mehr in einen dementsprechenden wirtschaftlichen Vorteil durch Rücklaufminimierung umzusetzen sein.

In Abhängigkeit von der Bodenzahl beträgt die Rücklaufmenge im Verhältnis zur Rohalkoholeinsatzmenge 2,6 : 1 für 55 Böden bis 1,5 : 1 für 125 Böden bezogen auf das Volumen.

Insbesondere stellt man mit dem geregelten Wassereintrag in die Trennkolonne und der daraus resultierenden Zusammensetzung des Kopfproduktes bzw. der Kopftemperatur ein Konzentrationsverhältnis von sec-Butylalkohol : Di-sec-butylether zwischen 0,7 : 1 bis 10 : 1, vorzugsweise zwischen 1 : 1 bis 2 : 1 ein.

Die Erfindung wird anhand der beigefügten Zeichnungen beispielsweise näher erläutert. Es zeigen:

Fig. 1     Dampf-Flüssigphasengleichgewicht des Systems sec-Butylalkohol-Wasser

Fig. 2     Darstellung der Löslichkeitsverhältnisse im ternären System Di-sec-butylether, sec-Butylalkohol und Wasser

Fig. 3     Fließbild des Verfahrens gemäß der Erfindung zur Abtrennung von azeotrop und leicht siedenden Verunreinigungen eines rohen wasserfreien sec-Butylalkohols

Fig. 4     Fließbild des Gesamtverfahrens gemäß der Erfindung zur destillativen Reinigung eines rohen wasserfreien sec-Butylalkohols

Fig. 5     Fließbild eines Teils des Verfahrens mit Abscheider gemäß einer ersten Ausführungsform der Erfindung

Fig. 6     Fließbild eines Teils des Verfahrens mit Abscheider gemäß einer zweiten Ausführungsform der Erfindung.

Die Untersuchung des Trennproblems gemäß der Aufgabenstellung führte zu den folgenden detaillierten Erkenntnissen:

## 1. Trennung sec-Butylalkohol - Wasser

Das in Fig. 1 wiedergegebene Dampf-Flüssigphasen-Gleichgewicht des Systems sec-Butylalkohol-Wasser mit

X-Achse = % sec-Butylalkohol in der Flüssigphase und

Y-Achse = % sec-Butylalkohol in der Dampfphase

zeigt, daß eine Trennung zwischen trockenem sec-Butylalkohol und dem Alkohol-Wasser-Azeotrop wenig Schwierigkeiten bereitet. Während einer Destillation erfolgt der Übergang vom Azeotrop zum trockenen Alkohol in einem Temperatursprung von 87,5° C auf 99,5° C. Bei einer kontinuierlichen Destillation macht sich dieser Temperatursprung im Bereich einiger Böden bemerkbar.

Es wurde gefunden, daß sich dieser Temperatursprung dazu eignet, über die Temperatur an einem geeigneten Boden in der Kolonne und einer von dort temperaturabhängig geführten Regelung der Energiezufuhr zum Verdampfer, ein konstantes Konzentrationsgleichgewicht in der Kolonne einzustellen. Als besonders geeigneten Richttemperatur hat sich eine Temperatur von 89° C bis 91° C bei Atmosphärendruck erwiesen. Bei einer Richttemperatur von zum Beispiel 90° C wird schon fünf Böden unterhalb des Temperaturführungsbodens ein Wassergehalt von <0,1 Gew.% im sec-Butylalkohol erreicht. Oberhalb des Temperaturführungsbodens wird schnell ein stabiles Konzentrationsgleichgewicht zwischen azeotroper Dampfphase und wasserhaltiger Flüssigphase auf den Destillationsböden eingestellt. Es ist dabei nicht möglich, eine Wasserkonzentration von mehr als etwa 20 Gew.% in den Flüssigphasen zu erreichen.

Bei dieser Art der Destillation wird das gesamte in die Destillationskolonne eingebrachte Wasser abgetrennt.

2. Abtrennung von Di-sec-butylether und Dimeren

Eine sinnvolle Abtrennung dieser Nebenprodukte von sec-Butylalkohol läßt sich nur mit Hilfe von Wasser als ternäre Azeotrope durchführen. Die Abtrennung solcher Produkte wird bei steigendem Siedepunkt der Produkte immer schwieriger, weil einmal damit die Anteile der abzutrennenden Produkte im ternären Azeotrop mit sec-Butylalkohol und Wasser immer geringer werden und zum anderen die Siedepunkte der ternären Azeotrope sich immer mehr dem Siedepunkt des binären Azeotrops sec-Butylalkohol - Wasser nähern.

Wasser ist aber nicht nur Hilfsmittel zur Ausbildung von ternären Azeotropen, sondern begünstigt auch die Abtrennung dieser Produkte, wenn es in der Flüssigphase im Gemisch mit sec-Butylalkohol vorliegt.

Aus diesem Grund wurde gemäß dem Stand der Technik durch eine große Wasserverdünnung der Dampfdruck von sec-Butylalkohol in der Flüssigphase der Kolonne verringert und dadurch die Flüchtigkeit der ternären Systeme aus dieser Flüssigphase erhöht. Gleichzeitig war zur Trennung auch eine ausreichende Anzahl von Destillationsböden und eine angemessene Rücklaufmenge erforderlich.

Wird nun eine Trocknung von sec-Butylalkohol im gleichen Trennschritt gefordert, so ist der Wassergehalt in den Flüssigphasen des Abtriebteils (wie schon beschrieben) auf etwa 20 % zu begrenzen. Zur Ergänzung der dadurch verringerten Kolonnentrennleistung (geringere relative Flüchtigkeit) müssen andere geeignete Maßnahmen ergriffen werden.

Es wurde gefunden, daß hierzu die Erhöhung der Dampfmenge im Abtriebsteil im Verhältnis zum Kolonnendurchsatz durch Rückführung einer entsprechenden Menge an Kopfprodukt an erster Stelle steht. Diese Erhöhung des Rücklaufverhältnisses hat energetisch gegenüber dem Verfahren des Standes der Technik kaum Bedeutung, da sich mit dem Wegfall der Wasserbelastung auch der Kolonnendurchsatz verringert und somit auf sec-Butylalkohol bezogen die Rücklaufverdampfung etwa im gleichen Rahmen bleibt.

Zweitens ist die Trennleistung der Kolonne durch die Anzahl der Abtriebsböden, auf denen ein Alkohol-Wasser-System vorliegt, im Verhältnis zur Dampf- bzw. Rücklaufmenge beeinflußbar.

Und drittens kann die Abtrennung von Ether und Dimeren durch eine niedrig gehaltene Konzentration dieser Produkte in der Kolonne dadurch begünstigt werden, daß einmal die Rohalkoholbeschickung in die Nähe des Kolonnenkopfes gelegt wird (z.B. 5 bis 10 Böden darunter), und daß zum anderen ein gewisser Überschuß an binärem sec-Butylalkohol - Wasser - Azeotrop in dem ternären Kopfproduktgemisch eingestellt wird.

Gelangen Ether und Dimere wegen unzureichender Trennleistung unter den wasserhaltigen Abtriebsteil der Kolonne, so sind diese Produktreste der Abtrennung entzogen. Gleichzeitig ist eine dementsprechende Verunreinigung des am Sumpf dieser Kolonne anfallenden sec-Butylalkohols mit diesen Produkten gegeben.

3. Abtrennung von tert-Butylalkohol und Isopropylalkohol

Die Abtrennung dieser beiden Alkohole bereitet aufgrund der Siededifferenzen ihrer binären Azeotrope TBA-Wasser 79,9 °C und IPA-Wasser 80,4 °C gegenüber dem sec-Butylalkohol - Wasser - Azeotrop (87,5 °C) keine besonderen Schwierigkeiten.

Es wurden jedoch gewisse Parallelen zum Verfahren des Standes der Technik gefunden, bei dem durch die hohe Wasserverdünnung die Abtrennung der wasserlöslichen leichtsiedenden Nebenprodukte erschwert und erst nach der Trocknung des sec-Butylalkohols möglich war.

Auch in einer für die Abtrennung von Ether, Dimeren und Wasser ausreichenden Trennkolonne wurden diese leichtsiedenden, aber wasserlöslichen Produkte teilweise mit dem kolonnenabwärts fließenden Strom durch die wäßrige Trennzone für Ether und Dimere durchgeschleppt.

Eine quantitative Abtrennung gelingt erst durch eine Nachtrennung aus dem wasserfreien sec-Butylalkohol. Dieses kann, wie in der US-PS 2 875 139 beschrieben, in einer zweiten Kolonne geschehen. Vorteilhafter ist es jedoch, diese Nachtrennung in der ersten Kolonne gleichzeitig durch einen entsprechend dimensionierten Abtriebsteil im unteren trockenen Kolonnenteil nach der Wasserabtrennung durchzuführen.

4. Zusammensetzung und Phasenzerfall des Kopfproduktes der Leichtsiederabtrennung

Bei der Abtrennung von leichtsiedenden und azeotrop leichtsiedenden Nebenprodukten aus sec-Butylalkohol unter Mithilfe von Wasser wird die Zusammensetzung des abzudestillierenden Kopfproduktes erstens von dem Verhältnis der Nebenprodukte in dem erzeugten Rohalkohol, zweitens aus der Summe der mit diesen Nebenprodukten möglichen binären und ternären Azeotrope und drittens aus dem Verhältnis zwischen dem im Kopfprodukt enthaltenen Wasser und dem der Kolonne zugeführten Wasser bestimmt. Andererseits kann dieses Wasserverhältnis und der Wasserhaushalt der Kolonne durch die Zusammensetzung des heterogenen Kopfproduktes und dem daraus resultierenden Lösungsgleichgewicht zwischen leichter organischer Phase und schwerer wäßriger Phase erheblich beeinflußt werden.

Wird z.B. der Kolonne bei einer durch die Menge der azeotrop siedenden Nebenprodukte vorgegebenen Verdampfung zu wenig Wasser zugeführt, so werden diese Produkte in steigendem Maße dem in der Kolonne eingestellten wasserhaltigen Abtriebsteil zunächst Wasser entziehen und schließlich zum Sumpf der Kolonne durchbrechen. Gleichzeitig wird sich der Gehalt an sec-Butylalkohol im Kopfprodukt verringern. Wird der Kolonne zu viel Wasser zugeführt, so werden mit jedem Teil überschüssiges Wasser 2,6 Teile sec-Butylalkohol in das Kopfprodukt getragen, was einen unerwünschten zusätzlichen Aufwand bei der Rückgewinnung des sec-Butylalkohols aus dem Kopfprodukt bedeutet.

Durch die in der Figur 2 dargestellte Gleichgewichtskurve A-B-C-D-E-F-G wird die Grenze der gegenseitigen Löslichkeit in dem System Di-sec-butylether (DSBE), sec-Butylalkohol (SBA) und Wasser $H_2O$) gebildet. Der Wassergehalt steigt und fällt mit der Konzentration an sec-Butylalkohol, so daß jede Änderung in dem Verhältnis von sec-Butylalkohol zu Di-sec-butylether auch eine Änderung der Wasserkonzentration mit sich bringt.

Die in der Tabelle III angeführten Zusammensetzungen zeigen anhand von zwei Modellbeispielen mögliche, aus der Zusammensetzung der Kopfprodukte resultierende Mengen- und Konzentrationsänderungen beim Phasenzerfall. Es wird deutlich, daß sich die Wassergehalte der azeotropen Kopfprodukte mit dem SBA-Gehalt weniger stark ändern als die Wassergehalte in den aus diesen Einstellungen anfallenden leichten organischen Phasen. In gleichem Maße ändert sich auch die Menge an schwerer wäßriger Phase, die z.B. bei Einsatz von wasserhaltigen Rohalkoholen abgezogen werden müßte.

Tabelle III

| | Kopfprodukt | | leichte Phase | | schwere Phase | |
|---|---|---|---|---|---|---|
| | % | Teile | % | Teile | % | Teile |
| Di-sec-butylether | 40,2 | 474 | 47,4 | 474 | - | - |
| sec-Butylalkohol | 39,8 | 470 | 46,0 | 460 | 5,5 | 10 |
| Wasser | 20,0 | 236 | 6,6 | 66 | 94,5 | 170 |
| Summe | 100 | 1180 | 100 | 1000 | 100 | 180 |

Verhältnis Di-sec-butylether : sec-Butylalkohol 1 : 1

| | Kopfprodukt | | leichte Phase | | schwere Phase | |
|---|---|---|---|---|---|---|
| | % | Teile | % | Teile | % | Teile |
| Di-sec-butylether | 25,9 | 300 | 30,0 | 300 | - | - |
| sec-Butylalkohol | 52,1 | 605 | 59,0 | 590 | 9 | 15 |
| Wasser | 22,0 | 255 | 11,0 | 110 | 91 | 145 |
| Summe | 100 | 1160 | 100 | 1000 | 100 | 160 |

Verhältnis Di-sec-butylether : sec-Butylalkohol 1 : 2

Diese Verhältnisse werden noch komplizierter, wenn die Zusammensetzung des azeotropen Kopfproduktes durch die Mengen und die Verhältnisse der Nebenprodukte wie tert-Butylalkohol oder Isopropylalkohol einerseits zu Di-sec-butylether und dimeren Nebenprodukten andererseits überlagert werden. Tert-Butylalkohol und Isopropylalkohol erhöhen die Wasserlöslichkeit im Sinne von sec-Butylalkohol. Dimere Nebenprodukte begünstigen die Wasserabscheidung wie Di-sec-butylether.

Ein weiterer zu berücksichtigender Eingriff in den Wasserhaushalt der Kolonne ist die Wassermenge, die mit wechselnden Mengen der im Rohalkohol enthaltenen Nebenprodukte und mit wechselnden Zusammensetzungen der dementsprechenden Teile der leichten organischen Phase ausgetragen wird.

Diese vereinfacht dargestellten, nur unzulänglich kontrollierbaren Vorgänge und gegenseitigen Abhängigkeiten bei der Einstellung des Wasserhaushaltes im Kolonnensystem werden noch komplizierter, wenn für die geforderte Trennaufgabe die Einhaltung eines konstanten thermodynamischen Gleichgewichts in der Trennkolonne Bedingung ist und als Kolonneneinsatz nur ein trockener Rohalkohol zur Verfügung steht.

Technische Lösungen, die den Wassergehalt im Rohalkohol oder den Wassergehalt in der Kolonne als Regelgröße benutzen, können hierbei nicht angewendet werden.

Es wurde überraschenderweise ein Verfahren gefunden, das die Nachteile der bekannten Verfahren beseitigt und auch bei der Reinigung eines trockenen sec-Butylalkohols die Einhaltung des Wasserhaushaltes im Kolonnensystem in dem gewünschten Sinne in einfacher und sicherer Weise ermöglicht.

Dazu wird bei einer für die Trennaufgabe fest eingestellten Rücklaufmenge praktisch das gesamte anfallende heterogene Kopfprodukt, unabhängig von seiner Zusammensetzung und dem daraus resultierenden Phasenzerfall, aus dem Rücklaufbehälter auf den obersten Boden der Kolonne zurückgeführt. Lediglich der aus den mit dem Rohalkohol eingetragenen Nebenprodukten und aus der Zusammensetzung des Kopfproduktes entstandene Zuwachs des Kopfproduktes wird als Teil der oberen organischen Phase als Überlauf aus dem Rücklaufbehälter abgezogen.

Zur Einhaltung des für die Trennaufgabe und für die Zusammensetzung des Kopfproduktes erforderlichen Wasserhaushaltes wird die Menge des in dem abgezogenen Nebenproduktstrom enthaltenen Wassers kontinuierlich als geregelte Frischwasserzufuhr über eine separate Leitung in den Rücklaufbehälter ergänzt.

Als Regelgröße für die Frischwasserzufuhr wird die Kopftemperatur der Trennkolonne herangezogen, deren Verlauf schon geringe Veränderungen in der für die Kolonneneinstellung gewünschten Zusammensetzung des Kopfproduktes bzw. der daraus anfallenden organischen Phase im Rücklaufbehälter erkennen läßt (siehe Tabelle IV).

Tabelle IV

| Kopfprodukt °C | 82,3 | 82,6 | 82,8 | 83,4 |
|---|---|---|---|---|
| Wasser Gew.% | 7,6 | 11,0 | 14,6 | 15,9 |
| Dimere Gew.% | 6,6 | 7,8 | 4,8 | 3,8 |
| Di-sec-butylether Gew.% | 37,6 | 22,6 | 17,1 | 15,0 |
| tert-Butylalkohol Gew.% | 18,4 | 20,8 | 27,5 | 24,0 |
| Isopropylalkohol Gew.% | 1,4 | 0,4 | 1,6 | 1,4 |
| sec-Butylalkohol Gew.% | 28,4 | 37,4 | 34,4 | 39,9 |
| Verhältnis | | | | |
| sec-Butylalkohol : Di-sec-butylether | 0,76 | 1,65 | 2,0 | 2,66 |

Die folgenden Beispiele erläutern die Erfindung.

Beispiel 1

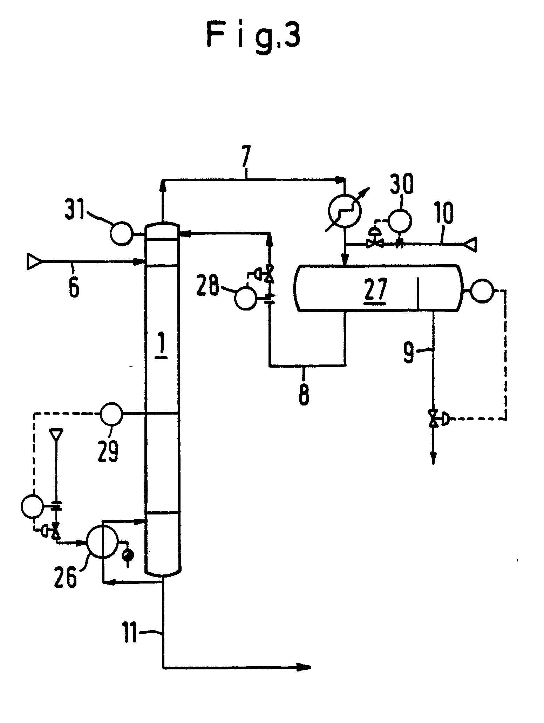

Das erfindungsgemäße Verfahren zur kontinuierlichen Abtrennung von leichtsiedenden und azeotrop siedenden Nebenprodukten von rohem trockenem sec-Butylalkohol wird mit Bezug auf das Fließbild der Fig. 3 nachfolgend beschrieben.

Ein praktisch wasserfreier, roher sec-Butylalkohol aus einem Direkthydratationsverfahren wurde über Leitung 6 kontinuierlich auf den 75. Boden von insgesamt 85 praktischen Böden der Kolonne 1, das heißt 10 Böden unter dem Kolonnenkopf, aufgegeben. Mit der Wahl des 75. Bodens wurde festgelegt, daß mit der Produktaufgabe in der Nähe des Kolonnenkopfes die Mehrzahl der Trennböden der Hauptaufgabe der Kolonne 1 als Abtriebskolonne dienen und nur ein kleiner Teil der Trennböden für eine begrenzte Rektifikation genutzt wird.

Durch Zugabe von Wasser über die Leitung 10 in den Rücklaufbehälter und Abscheider 27 und von dort über die Leitung 8 wurde zunächst im Kolonnensystem eine ausreichende Wasserkonzentration aufgebaut, die über die Temperaturregelung 29 die Einstellung eines der Richttemperatur von 90 °C ± 0,5 °C entsprechenden Wassergehaltes am 35. Boden erlaubte und die Einstellung eines wasserhaltigen azeotropen Kopfproduktes ermöglichte.

Abhängig von der Richttemperatur am 35. Boden wurde im Aufkocher 26 die zur Verdampfung der mit dem Rohalkohol eingebrachten Nebenprodukte und des über die Leitung 8 in die Kolonne geführten Rücklaufs notwendige Energie zugeführt.

Die zur Einstellung der geforderten Alkoholreinheit notwendige Trennleistung der Kolonne 1 wurde bei der vorgegebenen Zahl von insgesamt 85 praktischen Böden durch einen Verstärkerteil zwischen dem 75. Boden und dem Kolonnenkopf, einen wasserhaltigen Abtriebsteil von etwa 40 Böden (zwischen dem 35. und 75. Boden), einen trockenen Abtriebsteil von etwa 35 Böden (zwischen dem Kolonnensumpf und dem 35. Boden), ein darauf abgestimmtes Verhältnis von 1,9 Volumenteilen Rücklauf aus dem Abscheider 27 bezogen auf 1,0 Volumenteilen Rohalkoholeinsatz über Leitung 6 und einen ausreichenden Überschuß von sec-Butylalkohol im Kopfprodukt, der durch das Verhältnis von sec-Butylalkohol zu Di-sec-butylether zwischen 1 : 1 als Minimum und 2 : 1 als Maximum ausgewiesen wird, festgelegt.

Durch eine bei konstanter Rohalkoholzuführung mit dem Regelventil 28 fest eingestellte Rücklaufmenge über Strom 8 wurde neben einer relativ gleichmäßigen Energiezufuhr zum Aufkocher 26 auch eine ruhige Temperaturführung am Boden 35 und damit eine dauernde Einhaltung der Trennleistung in der Kolonne erreicht. Die Menge des als Strom 8 über das Regelventil 28 eingestellten Rücklaufs wurde ohne Rücksicht auf den Nebenprodukteintrag bzw. auf die Zusammensetzung des Kopfproduktes über Strom 7 oder des daraus resultierenden Phasenzerfalls im Abscheider 27 einzig und allein der in der Kolonne 1 geforderten und durch diesen Zwangsrücklauf ausgelösten Trennverschärfung im Abtriebsteil der Kolonne 1 angepaßt.

Das über die Leitung 7 nach der Kondensation im Abscheider 27 anfallende azeotrope Kopfprodukt entspricht in seiner Menge der Summe der mit dem heterogenen Rücklauf über Strom 8 eingebrachten Menge an organischer Phase + wässeriger Phase und der Menge der mit dem Rohalkohol eingebrachten Nebenprodukte.

Die Zusammensetzung des azeotropen Kopfproduktes und die der aus dem Phasenzerfall des Kopfproduktes resultierenden organischen Phase ergibt sich aus der Menge des mit dem heterogenen Rücklauf über Strom 8 in die Kolonne eingetragenen Wassers (als wässerige Phase und in der organischen Phase gelöst) in Abhängigkeit von der Menge und den azeotropen Eigenschaften der mit dem Rücklauf und dem Rohalkohol in die Kolonne eingebrachten und unter diesen Bedingungen leichtsiedenden Nebenprodukte.

Die aus dem Rohalkohol abgetrennten Nebenprodukte wurden in der sich aus der Zusammensetzung des Kopfproduktes ergebenden Zusammensetzung der leichten organischen Phase über Leitung 9 als Überlauf abgezogen und einer möglichen Weiterverarbeitung zugeführt.

Durch Leitung 9 wurde dem Kolonnensystem jedoch auch Wasser entzogen, dessen Menge von der Nebenproduktmenge und dem Wassergehalt aus dem Lösungsgleichgewicht im Abscheider 27 abhängt. Mit diesem Wasseraustrag über Leitung 9 stellte sich bei der trockenen Rohalkoholbeschickung zunehmend ein Wasserdefizit in der Kolonne ein. Damit wurde die für die Trennaufgabe geforderte sec-Butylalkohol-Konzentration bzw. das Mindestverhältnis zu Di-sec-butylether im Kopfprodukt unterschritten. Deshalb wurde dieser Wasseraustrag durch eine dementsprechende Wassermenge über die Leitung 10 und das Regelventil 30 in den Abscheider 27 ergänzt und von dort direkt mit dem heterogenen Rücklauf über Strom 8 auf den Kopf der Kolonne 1 zurückgeführt.

Die Menge des über das Regelventil 30 zugeführten Wassers muß sich in dem Maße ändern, wie sich die Menge der mit dem Rohalkohol eingebrachten Nebenprodukte und/oder die Zusammensetzung des Kopfproduktes und das daraus resultierende Lösungsgleichgewicht im Abscheider 27 ändert. Als erkennbares Maß für die Mengenänderung am Regelventil 30 wurde die über die Temperaturmessung 31 angezeigte Kopftemperatur herangezogen. Bei fallender Tendenz zur Soll-oder Richttemperatur wurde die Wassermenge über das Regelventil 30 erhöht und bei steigender Tendenz zur Richttemperatur verringert. Die Änderung der Richttemperatur und damit die der Zusammensetzung des Kopfproduktes geschah nicht sprunghaft, da sich auch das gesamte im Abscheider 27 enthaltene Produkt in seiner Zusammensetzung dem Kopfprodukt anpassen muß. Mit modernen Meß- und Regeleinrichtungen ist es möglich, die Abhängigkeit zwischen Kopftemperatur und Kopfproduktzusammensetzung einerseits und der zuzuregelnden Wassermenge andererseits automatisch zu steuern.

Die Gültigkeit der einer gewünschten Zusammensetzung entsprechenden Kopftemperatur, die ohne direkte Einflußnahme von Wasser nur durch das Mengenverhältnis von tert-Butylalkohol oder Isopropylalkohol zu Di-sec-butylether oder Dimere im Rohalkohol langfristig beeinflußt werden kann, wurde durch gelegentliche Kontrollanalyse der leichten organischen Phase in Leitung 9 überprüft.

Am Sumpf der Kolonne 1 wurde vorgereinigter, trockener sec-Butylalkohol über Leitung 11 abgezogen, aus dem die restlichen schwersiedenden Nebenprodukte in einer weiteren Kolonne abgetrennt und vom Sumpf der zweiten Kolonne abgeführt wurden.

Beispiele für verschiedene Destillationsbedingungen und den daraus resultierenden Nebenproduktkonzentrationen im Abtriebsteil der Kolonne bzw. im gereinigten sec-Butylalkohol als Sumpfabzug zeigt die Tabelle V.

## TABELLE V

Einfluß von Bodenzahl, Rücklaufverhältnis, sec-Butylalkohol-Konzentration im Kopfprodukt und Aufteilung des Abtriebsteils auf die Nebenproduktkonzentration im unteren Kolonnenteil.

| Beispiel | 1.1 | 1.2 | 1.3 | 1.4 | 1.5 | 1.6 | 1.7 | 1.8 | 1.9 | 1.10 | 1.11 |
|---|---|---|---|---|---|---|---|---|---|---|---|
| Gesamtzahl | 55 | 55 | 55 | 55 | 55 | 55 | 85 | 85 | 85 | 85 | 85 |
| Zulaufboden | 50 | 50 | 45 | 45 | 45 | 45 | 75 | 75 | 75 | 75 | 75 |
| Temperatur-führungsboden | 25 | 25 | 25 | 25 | 25 | 25 | 25 | 25 | 35 | 45 | 45 |
| Rohalkoholeinsatz 1/h | 1,5 | 1,5 | 1,5 | 1,0 | 1,0 | 1,0 | 1,0 | 1,0 | 1,0 | 1,0 | 1,0 |
| Rücklaufmenge 1/h | 2,6 | 2,6 | 2,6 | 2,6 | 2,6 | 2,6 | 2,1 | 1,9 | 1,9 | 1,9 | 1,7 |
| Verhältnis Rücklauf:Einsatz | 1,7 | 1,7 | 1,7 | 2,6 | 2,6 | 2,6 | 2,1 | 1,9 | 1,9 | 1,9 | 1,7 |
| Verhältnis SBA:DSBE im Kopfprodukt | 0,7 | 1,9 | 2,5 | 1,5 | 2,3 | 2,8 | 1,9 | 1,9 | 2,0 | 2,0 | 2,0 |
| Rohalkohol Gew.% TBA | 0,8 | 1,1 | 1,2 | 1,6 | 2,4 | 2,6 | 1,8 | 1,8 | 1,8 | 1,8 | 1,8 |
| Rohalkohol Gew.% DSBE | 1,3 | 1,2 | 1,1 | 5,9 | 2,6 | 0,9 | 1,3 | 1,3 | 1,3 | 1,3 | 1,3 |
| Boden 47 TBA | 9,9 | 3,4 | 1,4 | 0,73 | 1,24 | 1,8 |  |  | 0,73 | 0,79 | 1,95 |
| Boden 47 DSBE | 1,1 | 0,1 | 0,2 | 0,33 | 0,13 | 0,006 |  |  | <0,001 | <0,001 | <0,001 |
| Boden 42 TBA | 13,2 | 3,4 | 1,3 | 0,41 | 0,75 | 1,24 |  |  | 0,35 |  |  |
| Boden 42 DSBE | 0,6 | 0,03 | 0,04 | 0,05 | 0,02 | 0,002 |  |  |  |  |  |
| Boden 37 TBA | 19,9 | 3,2 | 1,1 | 0,11 | 0,24 | 0,45 | 0,14 | 0,25 | 0,044 | 0,018 | 0,031 |
| Boden 37 DSBE | 0,24 | 0,01 | 0,004 | <0,001 | 0,002 | <0,001 | 0,001 | <0,001 |  |  |  |
| Boden 28 TBA | 6,2 | 0,6 | 0,3 | 0,013 | 0,03 | 0,068 |  |  |  |  |  |
| Boden 28 DSBE | 0,25 | 0,01 | 0,002 |  | <0,001 |  |  |  |  |  |  |
| Boden 17 TBA | 1,8 | 0,13 | 0,06 |  |  |  |  |  |  |  |  |
| Boden 17 DSBE | 0,23 | 0,01 | 0,001 |  |  |  |  |  |  |  |  |
| Bdoen 13 TBA | 0,2 | 0,1 | 0,01 | 0,002 | 0,004 | 0,009 | 0,006 | 0,014 | 0,002 | 0,002 | <0,001 |
| Bdoen 13 DSBE | 0,2 | 0,01 | 0,001 |  |  |  |  |  |  |  |  |
| Boden 10 TBA |  |  |  | <0,001 | <0,001 | 0,001 | <0,001 | <0,001 | <0,001 | <0,001 | <0,001 |
| Boden 10 DSBE |  |  |  |  |  | <0,001 |  |  |  |  |  |
| Sumpfabzug TBA |  |  |  | <0,001 | <0,001 | <0,001 | <0,001 | <0,001 | <0,001 | <0,001 | <0,001 |
| Sumpfabzug DSBE |  |  |  |  |  |  |  |  |  |  |  |

## Beispiel 2

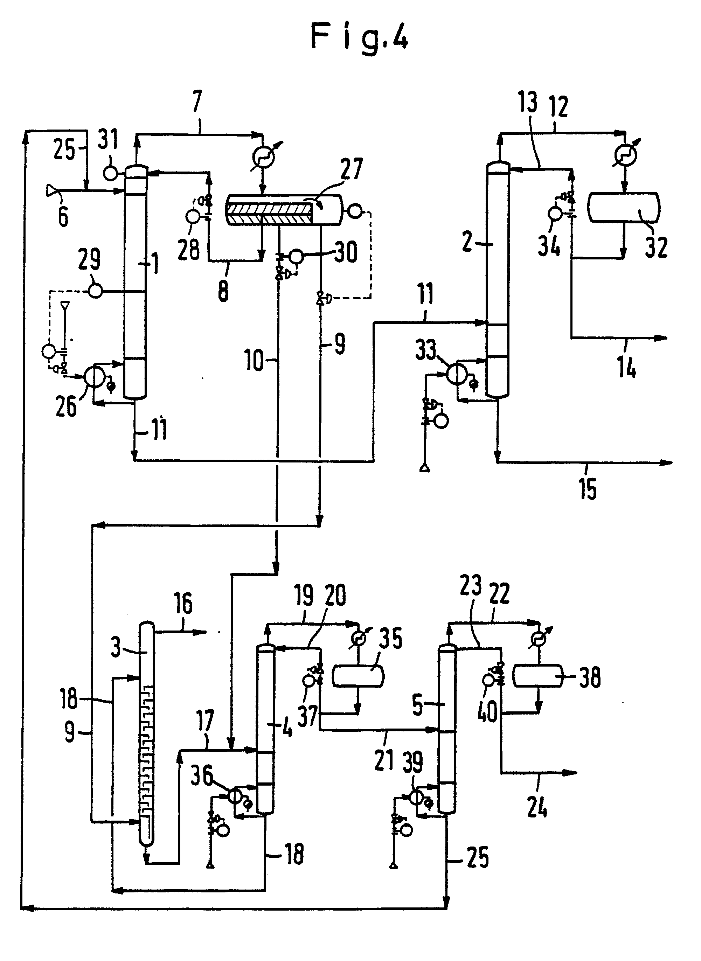

Gemäß einer weiteren Ausgestaltung des erfindungsgemäßen Verfahrens wird das gesamte Destillationsverfahren zur Reinigung eines rohen, wasserfreien sec-Butylalkohols, der durch Hydratation von n-Butenen in Gegenwart eines stark sauren Kationenaustauschers als Katalysator erhalten wurde, anhand des Fließschemas der Figur 4 erläutert.

Wie im Beispiel 1 wurde der trockene Rohalkohol über Leitung 6 zur Aufarbeitung eingesetzt, dem jedoch vor Eintritt in die Kolonne 1 über Leitung 25 ein wasserhaltiger sec-Butylalkohol, als Rückführstrom aus der Nebenproduktaufbereitung (vom Sumpf der Kolonne 5) anfallend, kontinuierlich zugesetzt wurde. Die Menge des über die Leitung 25 anfallenden Rückführstromes richtet sich im wesentlichen nach der aus dem Abscheider 27 über Leitung 9 abgezogenen Nebenproduktmenge und der darin enthaltenen sec-Butylalkoholkonzentration.

Der typische Wassergehalt des Alkohol-Wasser-Stroms in der Leitung 25 betrug etwa 30 Gew.%.

Die Destillationsbedingungen in der Kolonne 1 entsprechen denen im Beispiel 1. Der Destillationseinsatz aus Leitung 6 und Leitung 25 wurde auf den 75. Boden einer 85-bödigenKolonne aufgegeben. Durch eine automatisch geregelte Temperaturführung am 35. Boden wurde zwischen dem 35. Boden und dem 75. Boden ein wasserhaltiger Abtriebsteil von etwa 40 Böden und zwischen dem Kolonnensumpf und dem 35. Boden ein wasserfreier Abtriebsteil von etwa 35 Böden eingestellt.

Die mit dem Regelventil 28 eingestellte Rücklaufmenge über Leitung 8 betrug 1,9 Volumenteile bezogen auf 1,0 Volumenteile Rohalkoholeinsatz. Die mit dem Rohalkohol eingebrachten und über das Kopfprodukt ausgetragenen Nebenprodukte wurden als azeotropes Gemisch mit sec-Butylalkohol und Wasser über Leitung 9 aus dem Abscheider 27 abgeführt und in einer Nebenanlage zur Rückgewinnung des darin enthaltenen sec-Butylalkohols eingesetzt. Hier wurden im Extraktor 3 mit Wasser über Leitung 18, das im Kreislauf über die Kolonne 4 geführt wurde, alle wasserlöslichen Bestandteile, insbesondere sec-Butylalkohol, tert-Butylalkohol und Isopropylalkohol, aus dem Nebenproduktstrom 9 ausgewaschen. Über die Leitung 16 wurden alle wasserunlöslichen Nebenprodukte wie Di-sec-butylether und Dimere aus der Anlage abgeführt.

Aus dem alkoholhaltigen Waschwasserstrom 17 wurden in der Kolonne 4 die darin enthaltenen Alkohole als ein Gemenge der den Alkoholen entsprechenden binären Azeotrope mit Wasser über Kopf vom Wasserüberschuß abgetrennt und über die Leitung 21 abgeführt. Mit dem Regelventil 37 wurde über Leitung 20 mit einem Rücklauf im Verhältnis von etwa 1:1 bezogen auf den Strom der Leitung 21 eine ausreichende Trennung zwischen den Azeotropen und Wasser eingestellt.

Der azeotrope Alkohol-Wasser-Strom wurde über Leitung 21 in die Kolonne 5 überführt und destilliert. Die gegenüber dem bei 87,5° C siedenden Azeotrop sec-Butylalkohol-Wasser leichter siedenden Azeotrope von tert-Butylalkohol und Isopropylalkohol mit Wasser (kp 80° C) wurden über den Kopf der Kolonne abgetrennt und über Leitung 24 abgeführt. Mit dem Regelventil 40 wurde der Kolonne 5 über Leitung 23 soviel Rücklauf zugeführt, daß durch die dadurch gegebene Trennleistung der Kolonne der Verlust von sec-Butylalkohol über Leitung 24 bzw. die Rückführung leichtsiedender Nebenproduktalkohole über Leitung 25 vermieden bzw. in den gewünschten Grenzen eingehalten werden konnte.

Durch diese Maßnahmen wurde der gesamte, aus dem Abscheider 27 der Kolonne 1 abgezogene sec-Butylalkohol praktisch verlustfrei wieder in die Kolonne 1 zurückgeführt.

Wegen der begrenzten und durch die Anwesenheit von unpolaren Nebenprodukten noch verringerten Löslichkeit von Wasser war im Strom der Leitung 9 immer weniger Wasser auf sec-Butylalkohol bezogen enthalten, als in dem aus der azeotropen Zusammensetzung von sec-Butylalkohol und Wasser sich ergebenden Strom der Leitung 25. Diese Tatsache hatte ohne geeignete Maßnahmen zu einem lawinenartig ansteigenden Wasserüberschuß im System der Kolonne 1 geführt, da mehr Wasser im Kolonneneintrag mehr sec-Butylalkohol in das Kopfprodukt und damit in den Strom der Leitung 9 bringt und über die Rückgewinnung von mehr sec-Butylalkohol noch mehr Wasser über Leitung 25 in die Kolonne 1 eingebracht würde.

Im Rahmen des erfindungsgemäßen Verfahrens wurde jedoch ein einfaches technisches Konzept gefunden, das es gestattet, den Wasserhaushalt in der Kolonne 1 im umgekehrten Sinne wie im Beispiel 1 durch einen kontinuierlich geregelten Wasserabzug einzustellen und einzuhalten.

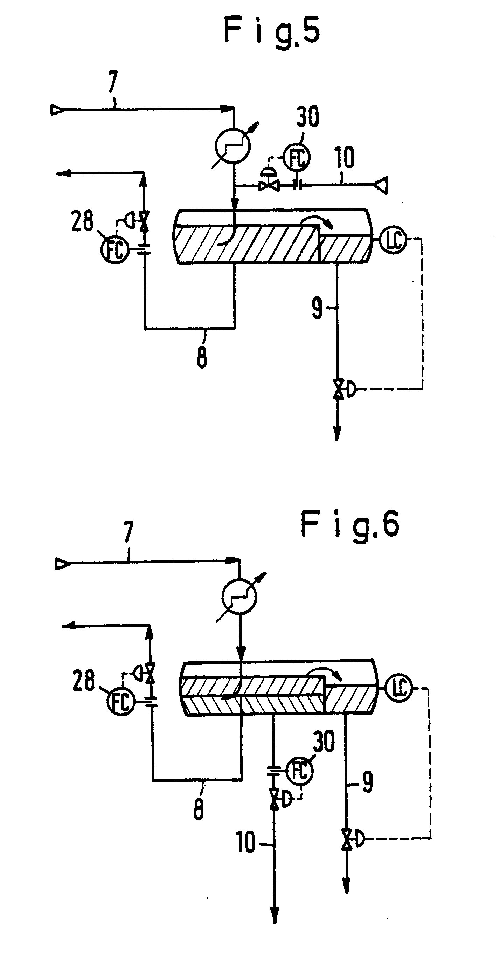

Dieses technische Konzept ist in vereinfachter Form als Figur 6 im Vergleich zu dem im Beispiel 1 angewandten Konzept der Figur 5 dargestellt.

Bei der Figur 5 handelt es sich um einen dem Stand der Technik entsprechenden konventionellen Abscheider, bei dem ein z. B. zylindrischer Behälter durch eine als Wehr ausgebildete Trennwand so in zwei Kammern geteilt ist, daß die Differenz an leichter organischer Phase aus der Zulaufmenge, Leitung 7, und aus der Abzugsmenge, Leitung 8, als Überlauf von der Zulaufkammer zur Ablaufkammer fließt und von dort über Leitung 9 abgezogen werden kann.

Die üblicherweise für eine Entwässerung gewünschte Abscheidung von schwerer Phase in der Zulaufkammer wird durch den direkten Abzug von leichter Phase aus der Zulaufkammer über Leitung 8 vermieden und dadurch die im Strom der Leitung 7 enthaltene und über Leitung 10 zugesetzte Wassermenge im

12

heterogenen Gemisch mit der leichten Phase sofort in die Kolonne 1 zurückgeführt.

Bei dem in Figur 6 dargestellten Konzept wurde die Leitung 8 als Einsteckrohr bis etwa in halber Höhe des Überlaufwehrs in die Zulaufkammer eingeführt. Dadurch ist einerseits die Abscheidung und der Abzug von schwerer, wasserreicher Phase möglich. Andererseits wird aber an der Trennlinie zwischen schwerer und leichter Phase, d. h. an der Oberkante des Einsteckrohres, die Menge des am Regelventil 28 eingestellten Rücklaufs zu Kolonne 1 ebenso direkt in der Zusammensetzung zurückgeführt, wie sie sich aus der Differenz an leichter Phase aus der Zulaufmenge, Leitung 7, und dem Überlauf zur Leitung 9 und aus der Differenz an schwerer Phase in der Zulaufmenge, Leitung 7, und der über Leitung 10 geregelt abgezogenen Wassermenge ergibt.

Mit dem geregelten Wasserabzug über Leitung 10 kann mit Bezug auf die Kopftemperatur als Indikator für die gewünschte Zusammensetzung des Kopfproduktes ein erhöhter Wassereintrag in die Kolonne 1 ausgeglichen und eine für die Trennung bevorzugte sec-Butylalkohol-Konzentration von 1:1 bis 2:1 Teile bezogen auf Di-sec-butylether eingestellt werden. Der im Lösungsgleichgewicht mit der leichten organischen Phase anfallende wässrige Strom 10 enthält u. a. sec-Butylalkohol gelöst und wird zur Rückgewinnung dieses Alkohols direkt über Leitung 17 dem Einsatz der Azeotropkolonne 4 zugeführt. Die im Strom der Leitung 10 enthaltene Wassermenge wird mit dem sec-Butylalkohol-Wasser-Azeotrop über Leitung 21 und dem Sumpfprodukt der Kolonne 5 über Leitung 25 im Kreislauf zur Kolonne 1 geführt.

Die Regelung am Regelventil 30 beeinflußt sich in einem überschaubaren Zeitraum dadurch selber, daß ein gleichmäßig eingestellter Wasserabzug über Leitung 10 auch eine gleichmäßige sec-Butylakohol-Konzentration in den Abzugsleitungen 9 und 10, gleichmäßige Betriebsbedingungen in den Apparaten 3 bis 5 und einen gleichmäßigen Wassereintrag über Leitung 25 in die Kolonne 1 verursacht.

Um bei einem hohen Anfall von tert-Butylalkohol bzw. Isopropylalkohol und durch das mit den Azeotropen über Leitung 24 ausgetragene Wasser (ca. 12 %) nicht in ein Wasserdefizit im Extraktor 3 zu gelangen, wird das Waschwasser in Leitung 18 in geeigneter Weise ergänzt.

Der wie im Beispiel 1 vorgereinigte sec-Butylalkohol wurde am Sumpf der Kolonne 1 als trockener Strom über Leitung 11 abgezogen und zur Abtrennung von schwersiedenden Nebenprodukten, bei denen der N-Butylalkohol mit 117,8° C dem Siedepunkt von sec-Butylalkohol mit 99,5° C am nächsten kommt, in die Kolonne 2 eingesetzt. Mit der über Leitung 15 abgezogenen Menge wurde eine begrenzte Anreicherung von Schwersiedern im Sumpf der Kolonne 2 und damit im Strom der Leitung 15 eingestellt. Durch diese begrenzte Anreicherung von Schwersiedern werden Temperaturbelastungen und Zersetzungen im Sumpfprodukt vermieden sowie die Trennanforderungen in der Kolonne 2 und die davon abhängige Rücklaufbelastung über Leitung 13 verringert. Als Maß für eine ausreichende Schwersiederabtrennung wurde eine n-Butylalkohol-Konzentration von max. 10 ppm in dem über die Leitung 14 anfallenden reinen sec-Butylalkohol angesehen.

Dieses Ergebnis wurde bei einer Anreicherung auf 5 % Schwersieder im Sumpf der Kolonne 2, bei 50 praktischen Böden zwischen dem Zulauf über Leitung 11 und dem obersten Boden und einem Rücklaufverhältnis von 0,5 erreicht.

Die im Strom der Leitung 15 enthaltene, auf die Produktion bezogen relativ kleine Menge an sec-Butylalkohol kann wahlweise verworfen oder an einer separaten Kolonne destilliert gewonnen oder dem Extraktoreinsatz (Leitung 9) zugesetzt und so über die Nebenproduktaufarbeitung verlustfrei im Kreis geführt werden. Die praktisch wasserunlöslichen schwersiedenden Nebenprodukte werden im letzten Fall mit dem Etherstrom über Leitung 16 ausgetragen.

Die Beschreibung des Gesamtverfahrens im Beispiel 2 wird ergänzt durch die in Tabelle VI aufgeführten Zusammensetzungen der wichtigsten Produktströme.

EP 0 304 770 B1

Tabelle VI

| Komponenten | in Leitung | | | | | | | |
|---|---|---|---|---|---|---|---|---|
| | 6 | 9 | 10 | 11 | 14 | 15 | 24 | 25 |
| Wasser | 0,02 | 11,0 | 89,4 | 0,01 | 0,01 | | 12,0 | 26,2 |
| Dimere | 1,17 | 5,8 | | <0,001 | < 0,001 | | | |
| Di-sec.-butylether | 4,73 | 24,1 | | <0,001 | < 0,001 | | | |
| tert.-Butylalkohol | 1,32 | 6,9 | 2,3 | <0,001 | < 0,001 | | 87,8 | 2,1 |
| sec.-Butylalkohol | 92,65 | 52,2 | 8,3 | 99,88 | > 99,98 | 93,9 | 0,2 | 71,7 |
| Schwersieder | 0,11 | | | 0,1 | < 0,001 | 6,1 | | |

**Patentansprüche**

1. Verfahren zur kontinuierlichen destillativen Reinigung von rohem sec-Butylalkohol, der durch katalytische Hydratisierung von n-Butenen bei erhöhter Temperatur und erhöhtem Druck und Abtrennung von nichtumgesetztem Olefin aus dem Reaktionsprodukt erhalten wurde, durch Einleitung des Rohalkohols in den oberen Teil einer Trennkolonne, Zuführung der zur Verdampfung notwendigen Energie durch einen Aufkocher am Bodenteil der Trennkolonne, Abdestillation der azeotrop und leichtsiedenden Nebenprodukte über Kopf in Gegenwart von Wasser und Abziehung des vorgereinigten sec-Butylalkohols am Sumpf der Trennkolonne und anschließende Abtrennung der schwersiedenden Nebenprodukte,
**dadurch gekennzeichnet,**
daß man zur Reinigung von praktisch wasserfreiem Rohalkohol dem obersten Boden der Trennkolonne die für die azeotrope Zusammensetzung des anfallenden Kopfprodukts erforderliche Wassermenge zuführt, und
durch Anlegen einer Richttemperatur im Bereich von 87,5° C bis 99,5° C an einen mittleren Boden der Trennkolonne (Temperaturführungsboden) das zugeführte Wasser in der Trennkolonne bis zu diesem Temperaturführungsboden herabführt,
den Rohalkohol auf einen Boden der Trennkolonne kurz unterhalb des Kolonnenkopfes (Zulaufboden) aufgibt, die unpolaren als ternäre Azeotrope mit SBA und Wasser leichtsiedenden Nebenprodukte in dem wasserenthaltendem Kolonnenteil zwischen Zulauf- und Temperaturführungsboden durch Extraktivdestillation azeotrop abtreibt, gleichzeitig die durch die Anwendung von Wasser in ihrer Abtrennung behinderten polaren Nebenprodukte in dem wasserfreien Kolonnenteil zwischen dem Temperaturführungsboden und dem Kolonnensumpf aus dem sec-Butylalkohol abtreibt, und diese Nebenprodukte gemeinsam in dem Kolonnenteil zwischen Zulaufboden und Kopf durch Rektifikation aufkonzentriert, das über Kopf anfallende Destillationsprodukt kondensiert und die erhaltenen zwei Phasen über einen Rücklaufbehälter in einem Strom direkt vom Boden des Rücklaufbehälters auf den obersten Boden der Trennkolonne zurückführt und lediglich den durch die Destillation des Rohalkohols bedingten Zuwachs an oberer leichter Phase als Nebenproduktstrom abzieht,
die zur Aufrechterhaltung der gewünschten azeotropen Zusammensetzung des Kopfprodukts erforderliche und in dem Nebenproduktstrom abgeführte Wassermenge durch Frischwasser ersetzt und dieses mit dem zweiphasigen Rücklauf dem obersten Boden der Trennkolonne zuführt, wobei man die Frischwasserzuführung anhand der Kopftemperatur der Trennkolonne regelt.

2. Verfahren nach Anspruch 1,
**dadurch gekennzeichnet,**
daß der Rücklaufbehälter so als Abscheider ausgebildet ist, daß man nicht nur die leichte Phase als Nebenpro duktstrom abführt und das zweiphasige Destillationsprodukt an der Trennlinie zwischen leichter und schwerer Phase abführt und auf den oberen Boden der Trennkolonne zurückführt, sondern auch aus dem unteren Teil des Behälters wäßrige Phase abzieht, daraus durch Azeotropdestillation sec-Butylalkohol-Wasser-Azeotrop gewinnt und dieses im Kreislauf zur Trennkolonne zurückführt.

3. Verfahren nach Anspruch 2,
**dadurch gekennzeichnet,**

14

daß man mit dem geregelten Wasserabzug einen erhöhten Wassereintrag in die Trennkolonne ausgleicht.

4. Verfahren nach einem der Ansprüche 1 bis 3,
   **dadurch gekennzeichnet,**
   daß man eine Trennkolonne mit 55 bis 125 Böden einsetzt, hiervon 5 bis 10 Böden oberhalb des Zulaufbodens als Rektifikationsteil anordnet und die anderen Böden durch Anlegen der Richttemperatur an den Temperatur führungsboden in einen wasserhaltigen (über dem Temperaturführungsboden) und einen trockenen Abtriebsteil (unter dem Temperaturführungsboden) aufteilt.

5. Verfahren nach Anspruch 4,
   **dadurch gekennzeichnet,**
   daß in Abhängigkeit von der Bodenzahl die Rücklaufmenge im Verhältnis zur Rohalkoholeinsatzmenge 2,6 : 1 für 55 Böden bis 1,5 : 1 für 125 Böden bezogen auf das Volumen beträgt.

6. Verfahren nach einem der vorhergehenden Ansprüche,
   **dadurch gekennzeichnet,**
   daß man mit dem geregelten Wassereintrag in die Trennkolonne und der daraus resultierenden Zusammensetzung des Kopfproduktes bzw. der Kopftemperatur ein Konzentrationsverhältnis von sec-Butylalkohol : Di-sec-butylether zwischen 0,7 : 1 bis 10 : 1, vorzugsweise zwischen 1 : 1 bis 2 : 1 einstellt.

7. Verfahren nach einem der vorhergehenden Ansprüche,
   **dadurch gekennzeichnet,**
   daß man als Richttemperatur eine Temperatur von 89 °C bis 91 °C bei Atmosphärendruck einstellt.

## Claims

1. A process for the continuous purifying distillation of crude sec-butyl alcohol obtained by catalytic hydration of n-butenes at elevated temperature and elevated pressure and by separation of unreacted olefin from the reaction product, wherein the crude alcohol is introduced to the upper part of a distillation column, the energy required for distillation is supplied by a reboiler at the bottom of the column, the azeotropically and low-boiling by-products are distilled off overhead in the presence of water, the prepurified sec-butyl alcohol is withdrawn at the bottom of the column and the high-boiling by-products are separated subsequently, said process comprising: feeding, for the purification of practically anhydrous crude alcohol, at the top of the column, the amount of water required for forming the azeotropic overhead product and maintaining the temperature in the middle of the column (temperature control tray) between 87.5 °C and 99.5 °C, whereby the water feed in the column is led down to said temperature control tray,
   feeding the crude alcohol to a tray shortly below the top of the column (feed tray), stripping azeotropically by extractive distillation, in the water-containing part of the column between feed tray and temperature control tray, the nonpolar by-products boiling at low temperatures as ternary azeotropes with SBA and water stripping at the same time, in the water-free part of the column between the temperature control tray and the bottom of the column, the polar by-products the separation of which is hampered due to the use of water, and concentrating said by-products altogether by rectification in the column section between feed tray and top of the column, condensing the distillation product obtained overhead and returning the two phases in a single stream from the bottom of the reflux drum directly to the top tray of the distillation column and withdrawing as a by-products stream only the increment in light upper phase resulting from the distillation of the crude alcohol, complementing by fresh water the water quantity required for maintaining the desired azeotropic composition of the overhead product and removed with the by-products stream, and feeding the fresh water together with the two-phase reflux to the top tray of the distillation column, the fresh water feed being controlled by the temperature at the top of the column.

2. A process according to claim 1 wherein the reflux drum is designed as a separator such that not only the light phase is removed as a by-products stream and the two-phase distillation product is removed at the light phase/heavy phase interface and is returned to the top tray of the distillation column, but also that from the lower part of said reflux drum aqueous phase is withdrawn, which, by azeotropic distillation, yields a sec-butyl alcohol/water azeotrope recycled to the distillation column.

3. A process according to claim 2 wherein the increased amount of water entrained in the distillation column is offset by the controlled removal of water.

4. A process according to any of the claims 1 to 3 wherein the distillation column contains 55 to 125 trays with 5 to 10 trays thereof above the feed tray as a rectifying section and with the remaining other trays functioning as a water-containing stripping section (above the temperature control tray) and a dry stripping section (below the temperature control tray) by maintaining a guide temperature at the temperature control point.

5. A process according to claim 4 wherein the volume ratio of reflux to the crude alcohol feed ranges from 2.6 : 1 for a 55-tray column down to 1.5 : 1 for a 125-tray column.

6. A process according to any of the preceding claims wherein water is supplied to the distillation column in an amount sufficient to maintain the ratio of sec-butyl alcohol to di-sec-butyl ether in the range of 0.7 : 1 to 10 : 1, preferably 1 : 1 to 2 : 1, as determined by the temperature at the top of the column.

7. A process according to any of the preceding claims wherein the guide temperature is within the range of 89 °C to 91 °C at atmospheric pressure.

**Revendications**

1. Procédé de purification continue par distillation d'alcool sec-butylique brut, qui a été obtenu par hydratation catalytique de n-butènes à température élevée et sous pression élevée et séparation du produit réactionnel de l'oléfine n'ayant pas réagi, par introduction de l'alcool brut dans la partie supérieure d'une colonne de séparation, apport de l'énergie nécessaire à la vaporisation par un rebouilleur se trouvant à la base de la colonne de séparation, élimination par distillation en tête des sous-produits bouillant en azéotrope et de bas point d'ébullition en présence d'eau et soutirage de l'alcool sec-butylique prépurifié à la base de la colonne de séparation, suivi de la séparation des sous-produits bouillant plus difficilement,
caractérisé en ce qu'on fait arriver la quantité d'eau nécessaire pour la composition de l'azéotrope du produit obtenu en tête au niveau du plateau supérieur de la colonne de séparation pour la purification d'alcool pur pratiquement anhydre, et en établissant une température directive dans la plage de 87,5°C à 99,5°C au niveau d'un plateau intermédiaire de la colonne de séparation (plateau de conduite de température), on fait descendre l'eau amenée dans la colonne de séparation jusqu'à ce plateau de conduite de température, on charge l'alcool brut sur un plateau de la colonne de séparation un peu en dessous de la tête de la colonne (plateau d'accès), on chasse par distillation azéotropique extractive les sous-produits non polaires de bas point d'ébullition formant des azéotropes ternaires avec l'alcool sec-butylique et l'eau dans la partie de colonne contenant de l'eau entre le plateau d'accès et le plateau de conduite de température, on chasse en même temps de l'alcool sec-butylique les sous-produits polaires dont la séparation est empêchée par l'utilisation d'eau dans la partie de colonne dépourvue d'eau entre le plateau de conduite de température et le fond de la colonne, et on concentre ces sous-produits conjointement dans la partie de colonne entre le plateau d'accès et la tête par rectification, on condense le produit de distillation passant en tête et on recycle les deux phases obtenues par l'intermédiaire d'un ballon de reflux en un seul courant directement du fond du ballon de reflux au plateau le plus haut de la colonne de séparation et on soutire comme courant de sous-produit simplement le surcroît de phase légère supérieure occasionné par la distillation de l'alcool brut, on remplace par de l'eau courante la quantité d'eau nécessaire pour l'entretien de la composition azéotropique désirée du produit de tête et éliminée dans le courant de sous-produit et on fait arriver cette eau courante avec le reflux à deux phases au plateau le plus haut de la colonne de séparation, en réglant l'amenée d'eau courante à l'aide de la température à la tête de la colonne de séparation.

2. Procédé suivant la revendication 1, caractérisé en ce que le ballon de reflux est réalisé à la manière d'un séparateur de façon telle que non seulement on élimine la phase légère comme courant de sous-produit et on élimine le produit de distillation à deux phases à l'interface entre la phase légère et la phase lourde et on le recycle au plateau supérieur de la colonne de séparation, mais on décharge aussi à la partie inférieure du ballon une phase aqueuse de laquelle on tire par distillation azéotropique un azéotrope formé d'alcool sec-butylique et d'eau et que l'on recycle à la colonne de séparation.

3.  Procédé suivant la revendication 2, caractérisé en ce qu'on compense une introduction d'eau élevée dans la colonne de séparation par l'enlèvement d'eau réglé.

4.  Procédé suivant l'une des revendications 1 à 3, caractérisé en ce qu'on utilise une colonne de séparation comportant 55 à 125 plateaux dont 5 à 10 plateaux sont disposés au-dessus du plateau d'accès comme partie de rectification et les autres plateaux se divisent, par l'établissement de la température directive au niveau du plateau de conduite de température, en une partie d'extrac-tion contenant de l'eau (au-dessus du plateau de conduite de température) et une partie d'extraction sèche (au dessous du plateau de conduite de température).

5.  Procédé suivant la revendication 4, caractérisé en ce que la quantité recyclée en rapport à la quantité introduite d'alcool brut s'élève, en fonction du nombre de plateaux, à 2,6 : 1 pour 55 plateaux et à 1,5 : 1 pour 125 plateaux, relativement au volume.

6.  Procédé suivant l'une des revendications précédentes, caractérisé en ce qu'on établit un rapport de concentration de l'alcool sec-butylique à l'éther de di-secbutyle compris entre 0,7 : 1 et 10 : 1, de préférence entre 1 : 1 et 2 : 1, par l'introduction réglée d'eau dans la colonne de séparation et par la composition du produit de tête ou par la température de tête qui en résulte.

7.  Procédé suivant l'une des revendications précédentes, caractérisé en ce qu'on établit comme température directive une température de 89 à 91 $^\circ$C à la pression atmosphérique.

# F i g.1

# Fig.2

# Fig.3

EP 0 304 770 B1

# Fig.4

21

# Fig.5

# Fig.6